# EUROPEAN PATENT SPECIFICATION

(11) **EP 3 511 006 B1**
(45) Date of publication and mention of the grant of the patent: **04.08.2021**
(21) Application number: 16912759.4
(22) Date of filing: 11.08.2016
(51) Int. Cl.: A61K 31/575, A61K 31/05, A61K 8/63, A61K 8/34, A61Q 7/00, A61P 17/14

(54) **COMPOSITION FOR PREVENTING, IMPROVING OR TREATING HAIR LOSS COMPRISING DEOXYCHOLIC ACID AS ACTIVE INGREDIENT**
ZUSAMMENSETZUNG ZUR PRÄVENTION, LINDERUNG ODER BEHANDLUNG VON HAARAUSFALL MIT DEOXYCHOLSÄURE ALS WIRKSTOFF
COMPOSITION POUR PRÉVENIR, AMÉLIORER OU TRAITER LA PERTE DES CHEVEUX, COMPRENANT DE L'ACIDE DÉSOXYCHOLIQUE COMME PRINCIPE ACTIF

(43) Date of publication of application: 17.07.2019
(73) Proprietor: Samsung Life Public Welfare Foundation, Seoul 04348 (KR); Solsion Biomedical Inc., Seoul 08594 (KR)
(72) Inventor: GWON, Hyeon Cheol, Seoul 06001 (KR); LEE, Dong Youn, Seoul 06291 (KR); JANG, Hyung Suk, Seoul 05507 (KR); KIM, Min Hee, Gangnam-gu Seoul 06351 (KR)
(74) Representative: Potter Clarkson
(86) International application number: PCT/KR2016/008859
(87) International publication number: WO 2018/030560

(56) References cited:
- WO-A1-2012/078649
- CA-A- 1 106 287
- FR-A1- 2 375 859
- KR-A- 20080 010 444
- KR-A- 20170 016 552
- KR-B1- 101 620 088
- US-A1- 2006 269 496
- US-B2- 8 258 146

## Description

### Technical Field

The present invention relates to a composition comprising deoxycholic acid as an effective ingredient for preventing, alleviating or treating hair loss.

### Background Art

Hair loss has been one of the greatest worries for someone with the recent increase of interest in appearance. Hair loss has been a concern, for the most part, in men in their 40's to 50's. Recently, alopecia has spread to youth in their 20's and 30's. An increasing population of women is also reported to suffer from hair loss nowadays. A variety of environmental factors including diet imbalance, harmful air, polluted drinking water, acidic rain, and stress aggravate hair loss in people at the present day. Therefore, many studies are ongoing for prevention and treatment of hair loss.

Each hair grows in cycles of various stages consisting of anagen in which hairs grow, catagen which signals the end of the active growth of a hair, with the regression of a hair bulb, Talogen in which the dermal papilla rests while a hair remains on the scalp, and exogen in which the dermal papilla starts to act or a new hair grows while an old hair is shed.

Anagen stage (2-7 years) is an active growth period of a hair and is divided into two stages of producing hair which grows outwards from the bulb into hair follicles; and generating hard keratins in the hair follicles. The hair continues to grow itself until the catagen stage.

Catagen stage (2-3 weeks) is a period during which hair growth ceases and the metabolism slow down while maintaining the shape of the hair. At this stage, keratin is not produced. The catagen stage accounts for 1 % of total hair growth. At this stage, the hair bulb shrinks and is divided into dermal papilla, and is surrounded by hair follicles and travels upwards. The cell division is ceases.

Talogen stage (3 months) is a period during which the dermal papilla shrinks and the hair follicle gradually shrinks, and the hair root crawls upwards and falls out. It is the period of hair loss until the next stage of growth and lasts for 3 to 4 months.

For a normal person, hairs in the anagen stages are predominant over those in the other stages whereas people with alopecia have hairs mostly in the Talogen stage, with a phenomenon of hair loss visible to a naked eye. As the hair loss progresses, the period of the anagen stage is shortened, resulting in the miniaturization of the hair. Accordingly, allowing the hair follicle in the Talogen stage to rapidly proceed to the anagen stage and prolonging the anagen stage are important for treatment of hair loss.

There are two kinds of medications approved for hair growth promotion by the FDA: minoxidil (transdermal agent) and finasteride (oral agent). Minoxidil was developed as an oral antihypertensive vasodilator medication and is now used as a topical agent for treatment of androgenic alopecia because hypertrichosis was observed in patients administered therewith. When applied to the scalp, minoxidil, which is a pyrimidine derivative, acts as a vasodilator to topically increase blood flow to activate keratinocytes whereby the progress of hair loss can be delayed and hairs in a new anagen stage appear. Thanks to this effect, minoxidil is widely used as a medication for hair loss treatment. Acting to inhibit type II 5α-reductase with the resultant effect of preventing hair loss and promoting hair regrowth, finasteride is the first oral medication approved for treatment of androgenic alopecia by the FDA in 1997 and at present, is used worldwide by about 2.6 million persons.

However, minoxidil has side effects including weight gain, edema, increased heart rates, angina pectoris, dermatitis, and itching. For finasteride, male sexual dysfunction was reported as a clinical case. These medications are thus limited in application or patients themselves are reluctant to use the medications. For the reason, the interest of consumers in the prevention of hair loss and the promotion of hair regrowth has turned toward safe agents derived from natural materials and therefore, active studies therefor are ongoing. Document CA 1 106 287 describes compositions of chenodeoxycholic acid and ursodeoxycholic acid that are used for scalp treatment and against hair loss.

### Detailed Description of the Invention

### Technical Problem

Leading to the present invention, intensive and thorough research into the development of an agent for prevention, alleviation or treatment of hair loss, conducted by the present inventors, resulted in the finding that deoxycholic acid, which is a bile acid, promotes the growth and proliferation of follicular cells to ultimately exhibit a hair regrowth effect.

Accordingly, an object of the present invention is to provide a pharmaceutical composition for preventing, alleviating or treating hair loss.

Described herein is a quasi-drug composition for preventing or alleviating hair loss.

A further object of the present invention is to provide the use of a composition in a cosmetic method for preventing or alleviating hair loss.

Other purposes and advantages of the present invention will become clarified by the following detailed description of the invention, claims, and drawings.

### Technical Solution

According to one aspect thereof, the present invention provides a pharmaceutical composition comprising deoxycholic acid as an effective ingredient for preventing, alleviating or treating hair loss.

Described herein is a quasi-drug composition comprising deoxycholic acid as an effective ingredient for preventing or alleviating hair loss.

According to a further aspect thereof, the present invention provides the use of a composition comprising deoxycholic acid as an effective ingredient in a cosmetic method for preventing or alleviating hair loss.

The present inventors have conducted research to develop an agent for treatment, alleviation, or treatment of hair loss. As a result, deoxycholic acid, a bile acid, is found to promote the growth and proliferation of follicular cells, thus ultimately exerting a hair regrowth effect.

Deoxycholic acid, which is used as the effective ingredient of the present invention, is a representative secondary bile acid, represented by the empirical formula C24H40O4, which is derived from cholic acid. Herein, deoxycholic acid and its salts are construed to fall within the scope of deoxycholic acid.

As used herein, the term "hair loss" refers to a loss of hair from the scalp or to hair thinning and can be interchangeable with alopecia. Examples of the hair loss include male-pattern hair loss (androgenic hair loss), alopecia areata, Talogen effluvium, drug-induced alopecia, mechanical alopecia, alopecia caused by a skin disorder, and cicatrical alopecia.

Therefore, the term "hair loss" should be understood to encompass all symptoms classified as hair loss in the art irrespective of whether the cause is direct or indirect.

According to an embodiment, the composition of the present invention is used for preventing hair loss or promoting hair growth or regrowth.

The term "preventing hair loss" means pertaining to preventing and suppressing alopecia as described above, the term "hair growth" means pertaining to prolonging the life of hair in the anagen stage to allow hairs to appear thick and healthy, and the term "hair regrowth" means pertaining to inducing or promoting the generation of hair in a new anagen stage. Therefore, the composition of the present invention may be used as a hair loss preventing agent, a hair growth agent, and/or a hair restorer.

The composition of the present invention may be formulated into suitable forms according to product modalities (pharmaceutical composition or cosmetic composition).

For example, the composition of the present invention may be in a form selected from the group consisting of a tonic, a spray, a cream, a lotion, an aerosol, oil, a solution, a suspension, a gel, an ointment, a paste, an emulsion, a liniment, a tablet, a capsule, a powder and a granule.

According to an embodiment, the composition of the present invention is an external skin preparation.

The composition of the present invention may comprise a different effective ingredient in addition to deoxycholic acid.

According to an embodiment, the different effective ingredient contained in the composition of the present invention is polyphenol.

The polyphenol available for the present invention may be exemplified by resveratrol, protocatechuic acid, gallic acid, p-hydroxybenzoic acid, caffeic acid, chlorogenic acid, cuomaric acid, ferulic acid, sinapic acid, pelargonidin, peonidin, delphinidin, malvidin, quercetin, myricetin, apigenin, luteolin, hesperetin, naringenin, eriodictyol, daidzein, genistein, glycitein, catechin and epicatechin.

According to a more particular embodiment, the polyphenol is resveratrol.

According to some embodiments, the composition of the present invention comprises 0.1-10 % by weight of deoxycholic acid.

The pharmaceutical composition of the present invention may comprise a pharmaceutically acceptable carrier.

The pharmaceutically acceptable carrier may be one typically utilized for formulation and examples thereof include, but are limited to, lactose, dextrose, sucrose, sorbitol, mannitol, starch, acacia gum, calcium phosphate, alginate, gelatin, calcium silicate, microcrystalline cellulose, polyvinylpyrrolidone, water, syrup, methyl cellulose, methylhydroxybenzoate, propylhydroxybenzoate, talc, magnesium stearate and mineral oil.

In addition to the above-mentioned ingredient, the pharmaceutical composition of the present invention may further comprise a lubricant, a humectant, a sweetener, a flavoring agent, an emulsifier, a suspending agent, a preservative, etc.

For concrete suitable pharmaceutically acceptable carriers and agents, reference may be made to Remington's Pharmaceutical Sciences (19th ed., 1995).

The pharmaceutical composition of the present invention may be administered orally or parenterally (e.g., transdermally).

The suitable dose of the pharmaceutical composition of the present invention may vary depending on various factors including the formulation method, the patient's age, weight, sex, the period of hair loss, the state of hair loss, the time of administration, the route of administration, excretion rate, and response sensitivity and a skilled physician with a moderate level of experience can easily determine and prescribe an effective dose for the desired prevention or treatment.

Additionally, the pharmaceutical composition may be prepared in a unit dose form or prepared to be contained in a multi-dose container by formulating with a pharmaceutically acceptable carrier and/or excipient, according to a method that a person skilled in the art can easily perform. In particular, the formulation may be in the form of a solution in oil or aqueous medium, a suspension, or an emulsion, or an extract, powders, granules, tablets, or capsules and may further include a dispersing agent or stabilizer.

According to the Korean Pharmaceutical Affairs Act, a quasi-drug refers to a product that has a weak action on the human body or does not directly act on the human body and which is not a tool or a machine, but an analogue thereof. A notice from the ministry of health and welfare in Korea classifies an external use agent for prevention of hair loss or promotion of hair regrowth as a quasi-drug.

In addition, according to the Korean cosmetics act, a cosmetic refers is defined as a product that cleans or beautifies the human body to enhance the charm or appearance of a person or is applied to the human body to maintain or enhance the health of the skin or hair and which has a slight effect on the human body.

The composition of the present invention may be prepared into any formulation form typically used in the art, for example, various external use agents such as paste, mousse, gel, liquid, emulsion, suspension, cream, ointment, sheet, aerosol, spray and liniment.

According to an embodiment, the composition of the present invention may be in the form of a hair shampoo, a hair rinse, a hair tonic, a hair conditioner, a hair essence, a hair lotion, a hair nutrient lotion, a hair cream, a hair nutrient cream, a hair moisturizing cream, a hair massage cream, a hair wax, a hair aerosol, a hair pack, a hair soap, a hair gel, a hair glaze, a hair mousse or a hair spray.

### Advantageous Effects

Features and advantages of the present invention are summarized as follows:
(a) The present invention provides composition comprising deoxycholic acid as an effective ingredient for preventing, alleviation, or treating hair loss.
(b) Because deoxychloic acid promotes the proliferation and growth of follicular cells to ultimately exhibit the effect of hair regrowth, the composition of the present invention can be used as a hair loss preventer, hair growth agent or hair restorer.

### Brief Description of the Drawings

FIG. 1 shows changes in the area of hair loss of group T (testosterone 10 mg/ml), group TD (testosterone 10 mg/ml + deoxycholic acid 1 mg/ml), and group TF (testosterone 10 mg/ml + finasteride 1 mg/ml).
FIG. 2 shows counts of follicular cells in group T (testosterone 10 mg/ml), group TD (testosterone 10 mg/ml + deoxycholic acid 1 mg/ml), and group TF (testosterone 10 mg/ml + finasteride 1 mg/ml).
FIG. 3 shows changes in the area of hair loss of group T (testosterone 10 mg/ml), group TR (testosterone 10 mg/ml + resveratrol 1 mg/ml), group TF (testosterone10 mg/ml + finasteride 1 mg/ml), and group TRF (testosterone 10 mg/ml + resveratrol 1 mg/ml + finasteride 1 mg/ml).

### Mode for Carrying Out the Invention

Hereinafter, the present invention will be described in detail with reference to examples. These examples are for illustrating the present invention more specifically.

### EXAMPLES

### EXPERIMENTAL MATERIALS AND METHODS

### Experimental Materials

Testosterone was purchased from Wako Pure Chemical Industry, and deoxycholic acid, finasteride, resveratrol, and N-Methyl-2-pyrrolidone (NMP) were Sigma Aldrich. Ethanol and glycerol was purchased from OCI Company Ltd. and Biosesang Company Ltd., respectively.

### Animal Preparation

B6CBAF1/j hybrid mice (5 weeks old, male, Charles River Japan) were acclimated for one week in a breeding room and then used from the time of 6 weeks after birth.

### Preparation of Agent

For use in an injection agent, a solvent was prepared to contain 15 % of ethanol, 5% of NMP, and 80 % of PBS. Solutions were prepared by mixing the solvent with the following solutes: group T (testosterone 10 mg/ml), group TD (testosterone 10 mg/ml + deoxycholic acid 1 mg/ml), group TR (testosterone 10 mg/ml + resveratrol 1 mg/ml), group TF (testosterone 10 mg/ml + finasteride 1 mg/ml), and group TRF (testosterone 10 mg/ml + resveratrol 1 mg/ml + finasteride 1 mg/ml).

### Animal Test

The animals were anesthetized with isoflurane through inhalation and injected subcutaneously at the neck with 100 µl of a testosterone solution (10 mg/ml) every day. From a couple of weeks, they were photographed along with a scale bar to quantify a hair loss area. After six weeks of testing, the mice were sacrificed by means of a CO2 incubator. Test areas of the skin were taken, fixed with formalin, and prepared into paraffin blocks. The blocks were horizontally sectioned and dyed with H&E (Hematoxylin & Eosin) to count follicles per mm².

### Test Result

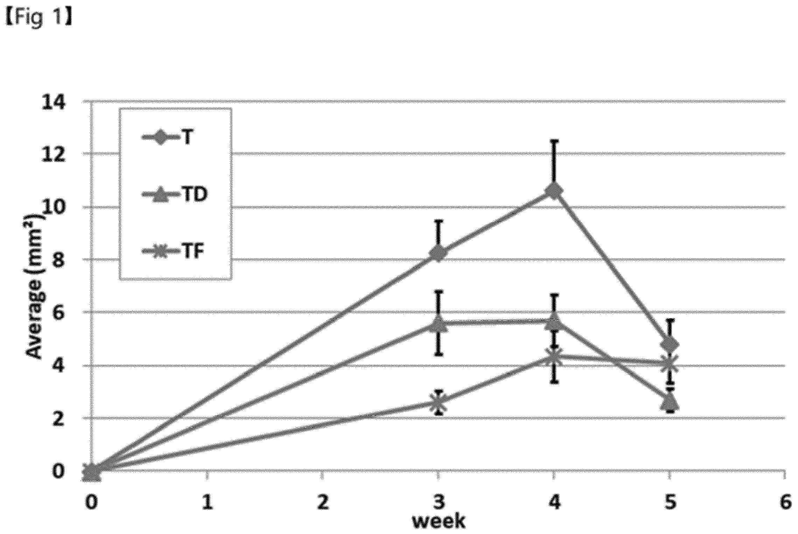

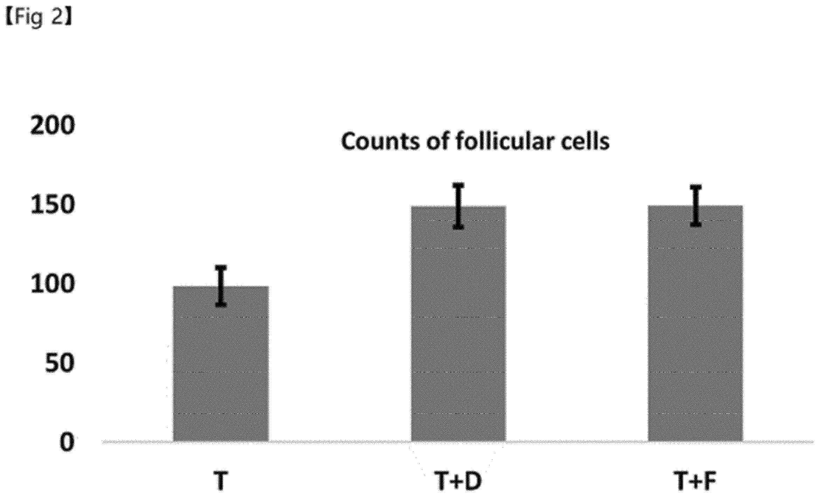

As shown in FIG. 1, the deoxycholic acid-treated group (group TD) was observed to remarkably reduce in hair loss area from week 3 after drug treatment, compared to the group treated with testosterone alone (group T). On week 4, a similar effect was found between the deoxycholic acid-treated group (group TD) and the finasteride-treated group (group TF) (FIG. 1). In addition, it can be seen in FIG. 2 that remarkable proliferation and growth of follicular cells were detected in the deoxycholic acid-treated group (group TD), compared to the group treated with testosterone alone (group T).

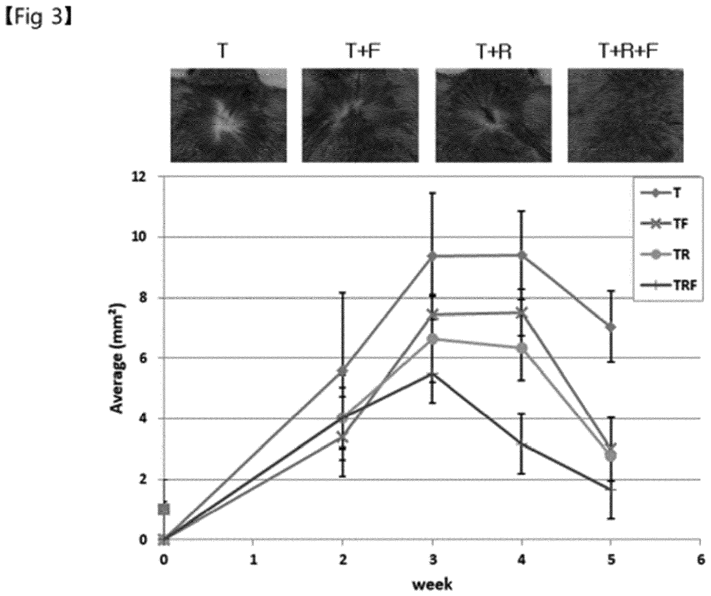

Even after two weeks, as shown in FIG. 3, the hair loss continued to increase in the group treated with testosterone alone (group T) while an increment in hair loss area was greatly reduced in the group treated with resveratrol (group TR) and with resveratrol and finasteride in combination (group TRF) (FIG. 3).

## Claims

1. A pharmaceutical composition comprising deoxycholic acid as an effective ingredient for use in a therapeutic method of preventing, alleviating or treating hair loss in a subject.

2. Use of a composition comprising deoxycholic acid as an effective ingredient in a cosmetic method for preventing or alleviating hair loss.

3. The composition for use according to claim 1, wherein the composition is used for preventing hair loss or promoting hair growth or hair regrowth.

4. The composition for use according to claim 1, wherein the composition is an external use agent.

5. The composition for use according to claim 1, wherein the composition has a form selected from the group consisting of a tonic, a spray, a cream, a lotion, an aerosol, an oil, a solution, a suspension, a gel, an ointment, a paste, an emulsion, a liniment, a tablet, a capsule, a powder, and a granule.

6. The composition for use according to claim 1, wherein the composition further comprises polyphenol as an effective ingredient.

7. The composition for use according to claim 6, wherein the polyphenol is resveratrol.

8. The use of a composition according to claim 2, wherein the composition is used for preventing hair loss or promoting hair growth or hair regrowth.

9. The use of a composition according to claim 2, wherein the composition is an external use agent.

10. The use of a composition according to claim 2, wherein the composition has a form selected from the group consisting of a tonic, a spray, a cream, a lotion, an aerosol, an oil, a solution, a suspension, a gel, an ointment, a paste, an emulsion, a liniment, a tablet, a capsule, a powder, and a granule.

11. The use of a composition according to claim 2, wherein the composition further comprises polyphenol as an effective ingredient.

12. The use of a composition according to claim 11, wherein the polyphenol is resveratrol.

## Patentansprüche

1. Pharmazeutische Zusammensetzung, die Desoxycholsäure als einen wirksamen Inhaltsstoff umfasst, zur Verwendung in einem therapeutischen Verfahren zum Vorbeugen, Lindern oder Behandeln von Haarausfall bei einem Subjekt.

2. Verwendung einer Zusammensetzung, die Desoxycholsäure als einen wirksamen Inhaltsstoff umfasst, in einem kosmetischen Verfahren zum Vorbeugen oder Lindern von Haarausfall.

3. Zusammensetzung zur Verwendung nach Anspruch 1, wobei die Zusammensetzung zum Vorbeugen von Haarausfall oder Fördern eines Wachsens von Haaren oder eines Nachwachsens von Haaren verwendet wird.

4. Zusammensetzung zur Verwendung nach Anspruch 1, wobei die Zusammensetzung ein Mittel für die äußere Verwendung ist.

5. Zusammensetzung zur Verwendung nach Anspruch 1, wobei die Zusammensetzung eine Form aufweist, die aus der Gruppe ausgewählt ist, die aus einem Tonikum, einem Spray, einer Creme, einer Lotion, einem Aerosol, einem Öl, einer Lösung, einer Suspension, einem Gel, einer Salbe, einer Paste, einer Emulsion, einem Einreibemittel, einer Tablette, einer Kapsel, einem Pulver und einem Granulat besteht.

6. Zusammensetzung zur Verwendung nach Anspruch 1, wobei die Zusammensetzung ferner Polyphenol als einen wirksamen Inhaltsstoff umfasst.

7. Zusammensetzung zur Verwendung nach Anspruch 6, wobei das Polyphenol Resveratrol ist.

8. Verwendung einer Zusammensetzung nach Anspruch 2, wobei die Zusammensetzung zum Vorbeugen von Haarausfall oder Fördern des Wachsens von Haaren oder des Nachwachsens von Haaren verwendet wird.

9. Verwendung einer Zusammensetzung nach Anspruch 2, wobei die Zusammensetzung ein Mittel für die äußere Verwendung ist.

10. Verwendung einer Zusammensetzung nach Anspruch 2, wobei die Zusammensetzung eine Form aufweist, die aus der Gruppe ausgewählt ist, die aus einem Tonikum, einem Spray, einer Creme, einer Lotion, einem Aerosol, einem Öl, einer Lösung, einer Suspension, einem Gel, einer Salbe, einer Paste, einer Emulsion, einem Einreibemittel, einer Tablette, einer Kapsel, einem Pulver und einem Granulat besteht.

11. Verwendung einer Zusammensetzung nach Anspruch 2, wobei die Zusammensetzung ferner Polyphenol als einen wirksamen Inhaltsstoff umfasst.

12. Verwendung einer Zusammensetzung nach Anspruch 11, wobei das Polyphenol Resveratrol ist.

## Revendications

1. Composition pharmaceutique comprenant de l'acide déoxycholique comme ingrédient efficace à utiliser dans un procédé thérapeutique de prévention, d'atténuation ou de traitement de la chute de cheveux chez un sujet.

2. Utilisation d'une composition comprenant de l'acide déoxycholique comme ingrédient efficace dans un procédé cosmétique destiné à prévenir ou à atténuer la chute de cheveux.

3. Composition destinée à être utilisée selon la revendication 1, la composition étant utilisée pour prévenir la chute de cheveux ou favoriser la croissance ou la repousse des cheveux.

4. Composition destinée à être utilisée selon la revendication 1, la composition étant un agent à usage externe.

5. Composition destinée à être utilisée selon la revendication 1, la composition ayant une forme choisie dans le groupe constitué par un tonique, un spray, une crème, une lotion, un aérosol, une huile, une solution, une suspension, un gel, une pommade, une pâte, une émulsion, un liniment, un comprimé, une capsule, une poudre et un granule.

6. Composition destinée à être utilisée selon la revendication 1, la composition comprenant en outre un polyphénol comme ingrédient efficace.

7. Composition destinée à être utilisée selon la revendication 6, le polyphénol étant le resvératrol.

8. Utilisation d'une composition selon la revendication 2, la composition étant utilisée pour prévenir la chute de cheveux ou favoriser la croissance ou la repousse des cheveux.

9. Utilisation d'une composition selon la revendication 2, la composition étant un agent à usage externe.

10. Utilisation d'une composition selon la revendication 2, la composition ayant une forme choisie dans le groupe constitué par un tonique, un spray, une crème, une lotion, un aérosol, une huile, une solution, une suspension, un gel, une pommade, une pâte, une émulsion, un liniment, un comprimé, une capsule, une poudre et un granule.

11. Utilisation d'une composition selon la revendication 2, la composition comprenant en outre un polyphénol comme ingrédient efficace.

12. Utilisation d'une composition selon la revendication 11, le polyphénol étant le resvératrol.
